# EUROPEAN PATENT APPLICATION

(11) **EP 4 052 651 A1**
(43) Date of publication of application: **07.09.2022**
(21) Application number: 21160768.4
(22) Date of filing: 04.03.2021
(51) Int. Cl.: A61B 6/00

(54) **IMAGE-BASED PLANNING OF TOMOGRAPHIC SCAN**

(71) Applicant: Koninklijke Philips N.V., 5656 AG Eindhoven (NL)
(72) Inventor: BERGTHOLDT, Martin, 5656 AE Eindhoven (NL); SENEGAS, Julien Thomas, 5656 AE Eindhoven (NL); DESHPANDE, Hrishikesh Narayanrao, 5656 AE Eindhoven (NL); SCHMITT, Holger, 5656 AE Eindhoven (NL); FRERKING, Lena Christina, 5656 AE Eindhoven (NL)
(74) Representative: Philips Intellectual Property & Standards

(57) **Abstract**

The present invention relates to a system (10) and related method for planning a tomographic image acquisition of an object to be imaged by a tomographic imaging scanner (20).The system comprises an input (12) for receiving a pre-scan image of the object and a camera system (14) for capturing at least one image of the object. The system comprises a processor (16) for determining corresponding image features in the at least one image captured by the camera system and the pre-scan image. The processor is further adapted to determine an image transformation that relates the corresponding image features to each other, such that the object as represented by the pre-scan image can be transformed by the transformation to the orientation, position and/or deformation of the object represented in the camera image. The processor automatically and/or interactively plans the image acquisition, in which the pre-scan image is used to determine parameters that include a scan range. The system also comprises an output (18) to output a signal representative of the determined plan.

## Description

### FIELD OF THE INVENTION

The present invention relates to the field of acquisition planning for a tomographic scanner, and, more specifically, the invention relates to a method and related device for accurately planning an image acquisition for such scanner based on a previously acquired image, e.g. a surview scan image.

### BACKGROUND OF THE INVENTION

In tomographic imaging, such as computed tomography (CT) and magnetic resonance imaging (MRI), scan parameters are often determined on the basis of a surview scan, which may also be referred to as a pilot, overview, scout, pre-scan or localizer scan. The parameters to be applied may depend on various factors, such as the intended purpose of the tomographic image acquisition, the position of the object to be imaged and its dimensions, e.g. a patient weight, body shape and/or volume. In addition to the volume to be imaged with respect to the scanner, e.g. determining a field of view and/or longitudinal length to be scanned, various other parameters may be affected by the properties of the specific object (e.g. patient) to be examined, such as beam settings for CT (peak beam energy, beam filtration, X-ray tube current, ...) and magnetic field gradients and/or radiofrequency (RF) pulse sequence parameters for MRI.

In computed tomography, the surview scan may typically consist of a projection image that is acquired by translating the patient (or another object to be imaged) through the CT gantry of the scanner while the X-ray source and detector remain stationary. Thus, a 2D projection image over the entire length of the patient is obtained. Alternatively, a low-dose (and therefore low-resolution, noisy and/or low-contrast) CT scan may be acquired to guide an operator in selecting a suitable imaging volume and/or other imaging parameters for a high-quality scan. Similarly, in MRI, a low-quality (and quick) overview scan may be used as localizer for planning a follow-up high-quality scan or sequence of scans.

Many approaches are known in the art to determine suitable scan parameters based on such surview image. For example, a CT operator may evaluate the surview image and may manually define a box on the image to indicate the region to be scanned. For this, the operator may rely on general guidelines to determine the suitable volume to be imaged, and possibly also other scan parameters, given the intended purpose of the scan (e.g. visualizing a specific body region, physiological and/or anatomical condition, and/or a specific suspected disease), for example by placing the plan box in a prescribed manner with respect to recognizable anatomical landmarks.

US 2016/012586 discloses a computer-assisted approach for determining a scan region of a subject to be scanned, in which a spatial transformation is determined by image registration of a surview image and a template image with respect to each other. The registration procedure is aided by initially relying on an element position indicator, e.g. of a reference geometrical or anatomical landmark (for example, the center of the brain), on the surview image, which has a known position in the template. A template scan region is defined with respect to the template image, such that the planned scan region can be determined by projecting the template scan region onto the overview image by means of the determined spatial transformation.

However, regardless of the method (manual or otherwise) that is applied to determine a suitable imaging volume and/or other imaging parameters, after the surview image has been acquired, the object to be scanned may have moved (particularly in case of a human or animal subject), such that the planned scanning operation would be suboptimal with respect to the changed position. Furthermore, it may be preferable to avoid capturing another surview image to replace the earlier captured image, even if the operator would be prompted to do so, e.g. after noticing a significant movement. For example, this could result in an undesirable loss of time, increased operating costs and, particularly in case of CT, an additional radiation dose to the imaged subject.

It is also known in the art, for example as described in US 2017/0316562, to determine a parameter of a contrast agent-assisted image acquisition by using camera imaging. In the approach described in that document, a camera image of the exterior of the patient is obtained to determine at least one body dimension, which is then used to determine the contrast agent protocol parameter, such as the amount of contrast agent to administer to the patient and/or the delay before acquiring diagnostic images using the scanner after administering the contrast agent.

### SUMMARY OF THE INVENTION

It is an object of embodiments of the present invention to provide in good, accurate and/or quick planning of a tomographic scan, for example to determine a desired scan region and/or other imaging parameters.

It is an advantage of embodiments of the present invention that an image acquisition can be planned on the basis of a previously acquired image, such as a surview scan, even if the object of interest, e.g. a patient, has moved since the acquisition of the previously acquired image.

It is an advantage of embodiments of the present invention that a loss of time and/or other operational resources due to recapturing a surview scan necessitated by a movement of the object to be imaged can be avoided.

It is an advantage of embodiments of the present invention that the acquisition of a surview image can be avoided if a previous scan of the object of interest is available, even if this previous scan was collected in a different scan session, e.g. possibly hours, days or even longer ago.

It is an advantage of embodiments of the present invention that missing important anatomical information in a diagnostic scan due to movement of a patient with respect to the imaging plan can be avoided.

It Is an advantage of embodiments of the present invention that three-dimensional (3D) imaging procedure planning on a 3D surview image can be easily combined with 3D camera data (e.g. range camera data) to correct the surview image and/or the imaging procedure plan for movement or repositioning of the imaged subject. For example, a rotation component around the longitudinal axis of a patient can be detected and compensated for by using a 3D surview in combination with 3D camera data. It is also an advantage that 3D surview images show a patient's spatial internal structure (but not necessarily at a high contrast and/or high resolution), such that an imaging procedure can be accurately planned. It is also an advantage that 3D surview images allow the body outline, i.e. the surface of the body, to be extracted easily, which can be correlated with 3D camera imaging data.

A system and method in accordance with embodiments of the present invention achieves the above objective.

In a first aspect, the present invention relates to a system for planning a tomographic image acquisition of an object to be imaged by a tomographic imaging scanner. The system comprises an input for receiving a pre-scan image of the object, a camera system for capturing at least one image of the object, a processor, and an output. The processor is adapted to determine corresponding image features in the at least one image captured by the camera system and the pre-scan image, to determine an image transformation that relates the corresponding image features to each other, such that the object as represented by the pre-scan image can be transformed by the transformation (or equivalently by its inverse) to the orientation, position and/or deformation of the object represented in the camera image, to automatically and/or interactively plan the tomographic image acquisition, in which the pre-scan image is used to determine parameters of the image acquisition that at least include a scan range in one dimension, and to output a signal, via the output, that is representative of the determined plan (or its associated parameters).

In a system in accordance with embodiments of the present invention, the camera system may comprise a range camera, such that the at least one image provided to the processor comprises three-dimensional information.

In a system in accordance with embodiments of the present invention, the camera system may comprise a plurality of cameras for imaging the object from a plurality of different viewpoints and a device to determine depth information from a plurality of images captured by the plurality of cameras, such that the at least one image provided to the processor comprises three-dimensional information.

A system in accordance with embodiments of the present invention may comprise a user interface, operably connected to the processor, to enable a user, by using the user interface, to interactively plan the image acquisition and/or to supervise and/or review an automatically generated plan.

In a system in accordance with embodiments of the present invention, the processor may be adapted to repeatedly perform the steps of capturing the at least one image with the camera system, determining corresponding image features and determining the image transformation, in which the pre-scan image and/or the imaging plan are repeatedly updated by the most recently determined transformation.

A system in accordance with embodiments of the present invention may comprise a storage device, wherein the input is adapted to retrieve the pre-scan image from the storage device.

A system in accordance with embodiments of the present invention may comprise the tomographic imaging scanner, in which the camera system is configured in a predetermined imaging geometry with respect to the tomographic imaging scanner.

In a system in accordance with embodiments of the present invention, the input may be operably connected to the tomographic imaging scanner to obtain the pre-scan image from the tomographic imaging scanner.

In a system in accordance with embodiments of the present invention, the tomographic imaging scanner may comprise a computed tomography scanner or a magnetic resonance imaging scanner.

A system in accordance with embodiments of the present invention may comprise a console to operate the tomographic imaging scanner and/or a workstation to prepare an imaging plan for the tomographic imaging scanner.

In a second aspect, the present invention relates to a method for planning an image acquisition of an object to be imaged by a tomographic imaging scanner. The method comprises obtaining a pre-scan image of the object, capturing at least one image of the object with a camera system that is configured in a predetermined imaging geometry with respect to the tomographic imaging scanner, determining corresponding image features in the at least one image captured by the camera system and pre-scan image, determining an image transformation that relates the corresponding image features to each other, such that the object as represented by the pre-scan image can be transformed by said transformation, or by its inverse, to the orientation, position and/or deformation of the object represented in the camera image, and planning the tomographic image acquisition, in which the pre-scan image is used to determine parameters of the image acquisition that at least include a scan range in one dimension.

The planning of the image acquisition comprises transforming the pre-scan image in accordance with the determined image transformation such that the position, orientation and/or deformation of the object in the pre-scan image corresponds to the position, orientation and/or deformation of the object as observed by the camera system, and/or transforming the determined parameters in accordance with the determined image transformation, such that said scan range as determined with respect to the pre-scan image is transformed to a same volume in the object as observed by the camera system.

In a method in accordance with embodiments of the present invention, capturing of the at least one image may comprise capturing an image comprising depth information, and obtaining the pre-scan image may comprise obtaining a three-dimensional tomographic scan image, e.g. a 3D tomographic surview scan image.

In a method in accordance with embodiments of the present invention, obtaining the pre-scan image may comprise retrieving the pre-scan image of the object from a storage device, where it was stored after being collected by the same or a different tomographic imaging scanner.

In a method in accordance with embodiments of the present invention, obtaining the pre-scan image may comprise acquiring the pre-scan image using said tomographic imaging scanner.

In a method in accordance with embodiments of the present invention, determining corresponding image features may comprise detecting a plurality of surface features of the surface of the object in respectively the pre-scan image and the camera image, and determining corresponding pairs of the detected surface features between said images by comparing a local surface geometry of the body surface around each detected surface feature.

In a method in accordance with embodiments of the present invention, determining corresponding image features may comprise using a constellation model of the relative locations of the detected image features to identify and remove spurious detected features.

A method in accordance with embodiments of the present invention may comprise executing the tomographic image acquisition using said tomographic imaging system in accordance with the determined parameters of said planning.

In a method in accordance with embodiments of the present invention, the steps of capturing the at least one image with the camera system, determining corresponding image features and determining the image transformation may be performed repeatedly, wherein the pre-scan image and/or the imaging plan are repeatedly updated by the most recently determined transformation.

A method in accordance with embodiments of the present invention may comprise alerting an operator when the determined transformation indicates a substantial movement of the object.

In a third aspect, the present invention relates to a computer program product for performing a method in accordance with embodiments of the second aspect of the present invention when executed on a computer.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

FIG 1 shows a method in accordance with embodiments of the present invention.
FIG 2 schematically illustrates a pre-scan image and a camera image, on which corresponding features can be identified in accordance with a method of embodiments of the present invention.
FIG 3 shows a system in accordance with embodiments of the present invention.
FIG 4 illustrates a computed tomography scanner in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for planning an image acquisition of an object to be imaged by a tomographic imaging scanner based on a pre-scan image of the object, e.g. at least one previously acquired projection and/or tomographic image.

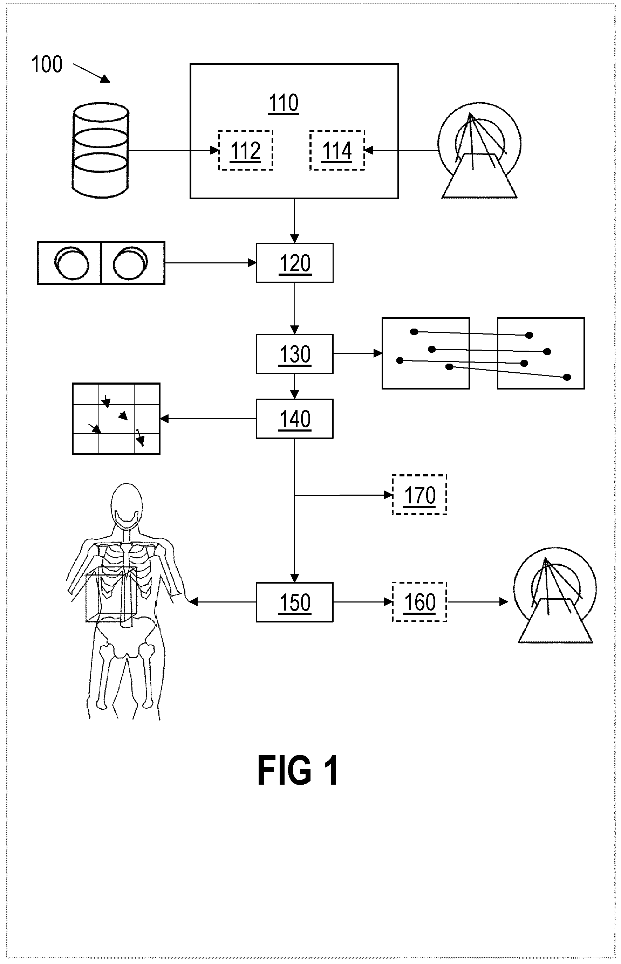

Referring to FIG 1, an example of a method 100 in accordance with embodiments of the present invention is shown. The method in accordance with embodiments of the present invention may be a computer-implemented method.

The method 100 is a method for planning an image acquisition, e.g. a tomographic (volumetric) image acquisition to visualize the internal structure of an object of interest, often a human or animal subject for diagnostic or research purposes. The planned image acquisition is to be acquired by a tomographic imaging scanner, such as a computed tomography (CT) or magnetic resonance imaging (MRI) scanner.

The method 100 comprises obtaining 110 a pre-scan image of the object under study to aid in the planning. The pre-scan image may be acquired by the same tomographic imaging scanner. The pre-scan image may comprise a projection image, a combination of projection images or a tomographic (volumetric) image. For example, as known in the art, the pre-scan image may be a surview (pilot; scout; localizer) image. The pre-scan image may be a two-dimensional projection (or cross-sectional) image, e.g. a 2D surview scan image, similar to a conventional projection X-ray image. As known in the art, such projection image may be acquired by translating the object with respect to the gantry of a CT scanner (e.g. using an automated translation couch) without rotating the gantry. However, preferably, the pre-scan image may be a three-dimensional tomographic image, e.g. a 3D surview scan image. An example of a 3D pre-scan is a low dose volumetric CT scan, which is typically unsuitable for diagnostic purposes due to the implied low image quality (e.g. lower signal to noise ratio, lower resolution and/or lower image contrast).

Ultra low dose 3D CT surview scans offer many advantages compared to conventional 2D surview scans. The geometry (e.g. anatomy) of the object can be discerned in detail without blending or obscuring information due to projective overlap. Furthermore, the beam projection geometry used for conventional 2D surview scans may distort the image, e.g. due to the fan shape of the beam, which could lead to inaccuracy in determining a precisely delineated region of interest to scan in detail.

Obtaining 110 the pre-scan image may comprise retrieving 112 the pre-scan image, of the same object, from a storage device, where it was stored after being collected in the past. Such pre-recorded pre-scan may be acquired by using the same tomographic imaging scanner, but this is not necessarily the case. The pre-stored image may be acquired by another tomographic imaging scanner of the same type, e.g. a different CT scanner, or may even be collected by a different tomographic imaging scanner type, e.g. by an MRI scanner where the planned procedure is intended for a CT scanner or vice versa. In some cases, it may even be preferable to use a cross-modality reference image as the pre-scan. For example, anatomical landmarks in the body, with respect to which a prescribed procedure is defined, may be easily recognizable on a CT pre-scan, where the feature of interest may be better visualized on a different modality, such as MRI.

Obtaining 110 the pre-scan image may also comprise acquiring 114 the pre-scan image using the tomographic imaging scanner (for which the procedure is planned). This is a common approach, in which the pre-scan image (e.g. a surview scan) is acquired in-situ by the scanner, to be followed by planning the procedure on the basis of the pre-scan image and subsequently performing the planned imaging operations.

However, where an in-situ collected pre-scan (see step 114) is typically of low quality, e.g. due to time and/or dose constraints, it is to be noted that, if images of the same object from a previous imaging procedure are available, it is a further advantage of reusing such previously acquired image (see step 112) that these images may have been collected at a higher resolution and/or better image quality than would typically be used for a mere surview. Nonetheless, the stored image may also be a surview scan obtained in a previous examination, such that no quality increase is necessarily implied compared to collecting a new surview scan in-situ. However, this does not negate the advantage of avoiding a loss of time and/or an increased radiation dose to the patient by reusing a previously acquired surview scan.

The method comprises capturing 120 at least one image with a camera system (configured in a known, e.g. predetermined, imaging geometry with respect to the tomographic imaging scanner) of the object to be scanned. The camera system may be an optical and/or infrared imaging system. Preferably, the camera system is adapted to collect depth information, e.g. depth information in addition to two-dimensional imaging. For example, the camera system may comprise a range camera, such as a time-of-flight depth imaging system, a structured light imaging system, a lidar system, or the like. Alternatively (or additionally), the camera system may comprise a plurality of cameras for imaging the object from different points of view, such that depth information can be inferred from correspondences in the images, e.g. a stereographic imaging system or generalization thereof that is suitable for more than two cameras. While a range camera may have the advantage of providing depth information, e.g. information about the distance of each image pixel (or a subset thereof or of a limited number of points in the image not excluded) along the optical path, in a direct or easily derivable form, it is known in the art that such information may also be provided by processing image information, e.g. by correlating multiple camera views, using a suitable prior-art algorithm. While the camera system may be adapted to collect depth information in addition to other image information on a pixel-by-pixel basis, such as light intensity and/or color or spectral information, this is not necessarily the case. For example, the camera system may adapted to collect a scalar image, in which the pixel values represent a distance to the imaged point along its optical path. As said, the camera system may collect vector-valued image information (directly or after suitable processing), in which such distance (range) information is supplemented by intensity and/or color and/or, spectral information. Nonetheless, embodiments in which the camera system captures merely intensity and/or color information without additional depth information is not necessarily excluded.

The method comprises determining 130 corresponding image features (e.g. at least two, e.g. preferably at least three, e.g. at least five, even more preferred at least ten of such image features) in the at least one image captured by the camera system and pre-scan image. For example, the image features may comprise salient image features of the imaged object, such as landmark features on the surface of the body of a patient. Such landmarks may comprise bony features, e.g. the frontal bone of the cranium, the nasal bone, the zygomatic arch, the mandible, the clavicles, the acromion process, the tubercle of the humerus, the sternum, the thoracic arch, the iliac crest of the pelvis, the patella and/or the tibial tuberosity, without limitation to these examples, and/or soft-tissues that influence the surface shape, e.g. muscles, fatty tissue, tendons, cartilage and/or ligaments that form recognizable features, such as surface depressions, furrows, folds and/or eminences. The landmarks may also relate to global anatomical features, e.g. joints, the neck, the head, the ribcage (or parts thereof), geometrical centers of body regions (e.g. of the head, the trunk, the extremities, ...), etcetera. The image features may also comprise features that are not necessarily point-like in nature, e.g. a surface model of (at least part of) the body surface and/or a descriptor(s) of a line(s), e.g. a longitudinal midline of the trunk.

While specific points in the images may be detected, which have an a priori known correspondence based on a specific method of detection for each type of point, this is not necessarily the case. For example, a plurality of points may be detected in the camera image and the pre-scan image based on more generic properties, such as local maxima/minima on the surface (e.g. a top of a protrusion or bottom of a depression), saddle points, centers of folds and/or bulges and the like. As discussed further hereinbelow, points that are detected using geometrical local properties of the surface of the object may be matched to each other, i.e. in the camera image and the pre-scan image, to establish the correspondence.

Determining 130 these corresponding image features may comprise determining a model of the surface of the object, e.g. of a patient's body, from the pre-scan image. In case of a 2D pre-scan image, a model of the generic shape of the object may be matched to the projection image information, whereas a surface model can be easily constructed from a 3D pre-scan image, e.g. using an image segmentation and surface extraction method, examples of which are well-known in the art. As another example, if the pre-scan image is a 2D X-ray projection image, the pixel intensity through non-bony regions may be used to, approximately, estimate the thickness of the body along the projection ray. This allows an estimation of an approximate surface model, e.g. particularly when combined with a parametric model of the body shape. Furthermore, even if only 2D camera images and 2D pre-scan images are used, a method in accordance with embodiments can still be used to detect a uniform displacement and/or rotation in the image plane.

It is to be noted that the image features may merely refer to the extracted surfaces (or a selected region thereof) as such. For example, methods are known in the art to map a surface specification onto another surface specification, e.g. in which each surface is defined in terms of a suitable representation, e.g. a surface mesh. Even though such methods might be prone to overfitting, a suitable regularization method may be applied to enforce a more simple mapping pattern, i.e. to avoid unrealistic or infeasible mappings.

The method further comprises determining 140 an image transformation that relates the corresponding image features to each other, such that the object as represented by the pre-scan image can be transformed to the orientation, position and/or deformation thereof represented by the camera image. This image transformation relating the corresponding image features does not necessarily match each feature exactly to its corresponding feature, e.g. the transformation may be described by a plurality of parameters that do not allow for an exact, but only an approximate, match for all of the features collectively and/or (hard and/or soft) constraints may be taken into account to avoid or reduce the selection of unrealistic transformations. The image transformation may comprise a rigid coordinate transformation, e.g. a translation in two or more orthogonal directions and/or a rotation around two or more orthogonal directions, and/an affine transformation and/or a non-linear deformation, e.g. using an interpolated grid of displacement vectors (e.g. a B-spline deformation map, thin plate splines, ...) and/or another non-linear deformation (warping) model.

Determining 140 the image transformation may comprise the estimation of parameters of the image transformation (e.g. displacement vectors, spline parameters, coordinate transformation matrix elements, ...) by an optimization algorithm, e.g. minimizing a suitable cost function (or maximizing a suitable objective function) indicative of the positional and/or orientational match of the corresponding image features after applying the image transformation to either the pre-scan or the camera image (a scheme to combine partial transformations of both images not excluded) in accordance with the transformation parameters under estimation. As is known, such optimization may further take one or more constraints into account, e.g. to ensure a desired level of smoothness of a non-rigid transformation or other feasibility criteria.

For example, a plurality of corresponding image features, e.g. salient points, may be detected in the camera image as well as in the pre-scan image. The local surface geometry around these image features may be used to detect correspondences of pairs of features between the camera image and the pre-scan image. Furthermore, a constellation model for the relative locations of the image features may be used to refine the putative corresponding features, e.g. to reject false positive matches identified by similar local surface geometries. It will be understood that the method may also comprise a joint optimization of local matching and global constellation matching, an iterative approach of alternating both steps, a pyramidal approach in which initially a higher importance is given to the local matching to end in a higher weight attributed to the constellation matching, or variations thereof. Once a good set of correspondences is established, it will be appreciated that determining a suitable image transformation may be as straightforward as fitting parameters of the class of transformations under consideration to the corresponding points. Furthermore, in case specific features are detected based on highly selective criteria, e.g. a saddle point near the center of the frontal aspect of the trunk to detect the xiphoid process, detecting such set of good correspondences between the images may be particularly easy as well, avoiding the need of a more intricate matching, e.g. based on local surface geometry, and false positive rejection, e.g. based on constellation matching, as described hereinabove.

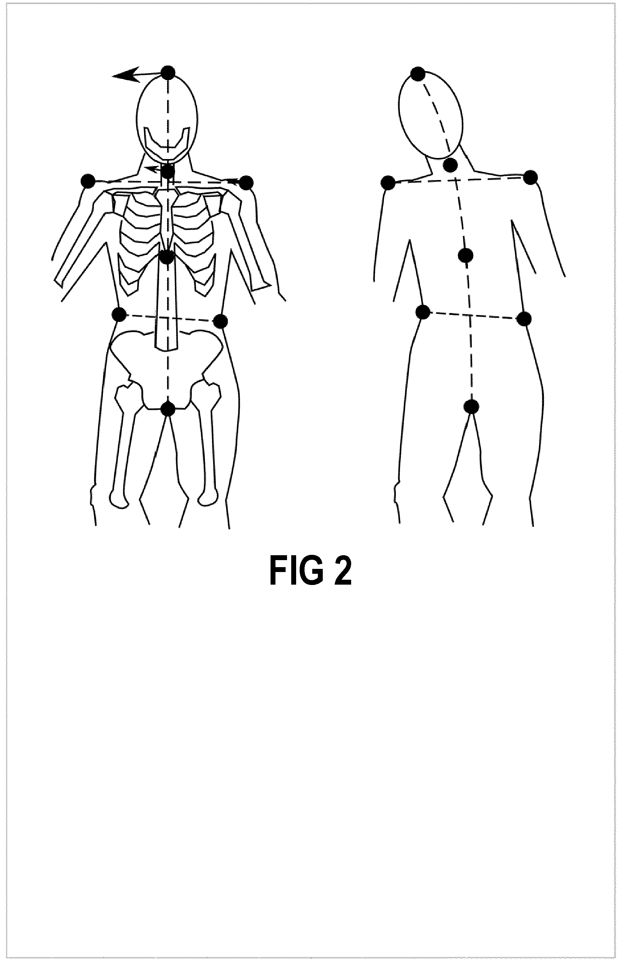

As an example, FIG 2 shows, to the left, a schematic illustration of a pre-scan image. This pre-scan image could be an anterior-posterior projection radiograph. To the right, a camera image is schematically illustrated. A contour of the body can be easily extracted from the pre-scan image, or, where the pre-scan image is a volumetric image, the exterior surface of the body. On this extracted contour/surface, salient features may be detected, e.g. by specifically identifying specific anatomical landmarks or by detecting geometrical features of the contour/surface. For example, such geometrical features could include peaks of protrusions, centers of ridges, centers of folds, saddle points, centers of substantially flat regions, and the like. In this example, without limitation thereto, the top of the cranium, the inferior point of the inguinal region, the top of the deltoid bulges, the center of the trunk and the points where the waistline is narrowest are indicated as illustrative image features. Additionally or alternative, some (or all) features may not be point features, e.g. the frontal centerline of the trunk may be detected as a line feature. Likewise, a similar feature detection approach may be applied to the camera image. Even though this simple example merely shows features that can be detected on the basis of a body outline, it is to be noted that the detection of features may be carried out particularly well when the camera image comprises depth information, e.g. as may be provided by a range camera.

Thus, correspondences between pairs of features, one element of the pair in the pre-scan image and the other in the camera image, can be detected (if not already implied by detecting specific landmark points), and displacement vectors can be determined to map the features of one of the images onto the corresponding features in the other image. This is illustrated by the displacement vectors depicted in the illustrative pre-scan image of FIG 2. These displacement vectors can be used to determine a image transformation, e.g. by imposing a (2D or 3D) grid of nodes onto the image, determining for each node an associated displacement, which may be optimized until interpolation of the displacement field over the entire image achieves a suitable mapping of the corresponding image features onto each other. It is to be noted that many alternative approaches are known in the art to describe and determine an image transformation, which may equally be applied.

The method 100 further comprises planning 150 the tomographic image acquisition, in which the pre-scan image is used to determine parameters of the image acquisition that at least include a scan range in one dimension, e.g. a scan range or (2D or 3D) bounding box to be scanned. This may also include selecting an orientation of the scan volume, e.g. rotating the bounding box around one or more axes to select a volume to be imaged. This may also include determining other parameters, such as X-ray beam properties, detector settings and the like.

Planning the acquisition may comprise providing a user interface to an operator to interactively define the parameters to be used on the basis of the pre-scan image, e.g. allowing the operator to draw a box to be scanned on the pre-scan image. Planning the acquisition may also comprise an automated procedure, in which the parameters are determined based on the pre-scan image, e.g. dependent on a selected type of imaging procedure. A combination of an automatic and manual planning may also be provided, e.g. in which an automatic selection is made that can be refined interactively.

Planning the acquisition 150 comprises transforming the pre-scan image in accordance with the determined image transformation such that the position, orientation and/or non-linear shape (deformation) of the object in the pre-scan image corresponds to the position, orientation and/or non-linear shape of the object as observed by the camera system. Alternatively, or additionally, planning 150 the acquisition may comprise transforming the determined parameters, such as a scan range or bounding box, in accordance with the determined image transformation, such that a movement of the object, as observed by the camera system with respect to the initial position/orientation/deformation in the pre-scan image, is compensated.

The method may comprise executing 160 the tomographic image acquisition in accordance with the determined parameters in the planning 150 step. Thus, in accordance with some embodiments, the operator may select (or supervise the selection) of imaging parameters on the basis of the initial pre-scan image, which are then automatically corrected for movement of the imaged object/subject before executing the image acquisition. In accordance with some embodiments, the pre-scan image may be corrected for the movement, such that the operator can plan (or supervise the planning) the procedure based on the current position/orientation/deformation of the object/subject to be imaged.

A method 100 in accordance with embodiments may perform the steps of capturing 120 the image with the camera system, determining 130 corresponding image features and determining 140 the image transformation repeatedly, e.g. such that the pre-scan image and/or the imaging plan can be repeatedly updated to conform to the most recent known position/orientation/deformation of the object to be examined.

Thus, planning of the imaging procedure can be carried out on a warped (preferably 3D) surview image that conforms to the present spatial configuration of the subject to be imaged. If a plan has already been drawn up, the plan can be warped to compensate for the present spatial configuration. If during an examination, e.g. between acquiring the pre-scan and executing the planned image acquisition, the object (e.g. patient) moves or moves again, the plan can be transformed to compensate. Furthermore, as already mentioned hereinabove, the camera system has a known spatial relation to the tomographic imaging scanner (i.e. is calibrated with respect to the CT/MRI coordinate system), such that the transformation can map coordinates directly into to coordinate space that is relevant for the scanner operation, or an additional known and predetermined transformation may be applied before execution. Therefore, a diagnostic scan with a planned reconstruction region (volume) can proceed without losing additional time for re-scanning or re-planning in case of patient movement.

An additional advantage of embodiments of the present invention is that a prior tomographic (e.g. CT or MRI) scan of the subject can be used to plan a new procedure. Thus, instead of a 3D surview scan, a prior 3D CT scan (which could be an earlier acquired 3D surview, but could also be a high quality diagnostic scan) of the same subject can be retrieved, assuming that subject's morphology has not changed significantly. This 3D scan can then be transformed, e.g. warped, using a surface and/or landmark feature correspondence approach as detailed hereinabove.

A method 100 in accordance with embodiments of the present invention may also comprise alerting 170 an operator, e.g. using an audio and/or visual cue, when the determined transformation exceeds a predetermined magnitude metric, e.g. when substantial movement is detected.

A method 100 in accordance with embodiments of the present invention may also comprise detecting a patient pose based on the detected image features, e.g. a spatial configuration of the detected image features, and using this pose information in a user interface. For example, a selection list of available procedures may be pruned based on the detected pose, e.g. to remove options that are incompatible with the detected pose. This may also (additionally or alternatively) include alerting an operator that the pre-scan image would require extensive warping to conform to the current pose, e.g. that it might be advisable to recapture another pre-scan image. It is also to be noted that the latter does not necessarily require the detection of a pose, e.g. determining a pose from a finite set of possible, general poses. For example, an average magnitude, a measure of dispersion (e.g. variance), a maximum or other suitable metric of the determined transformation may be compared to a threshold to determine whether an operator should be alerted that it might be advisable to recapture a new pre-scan image.

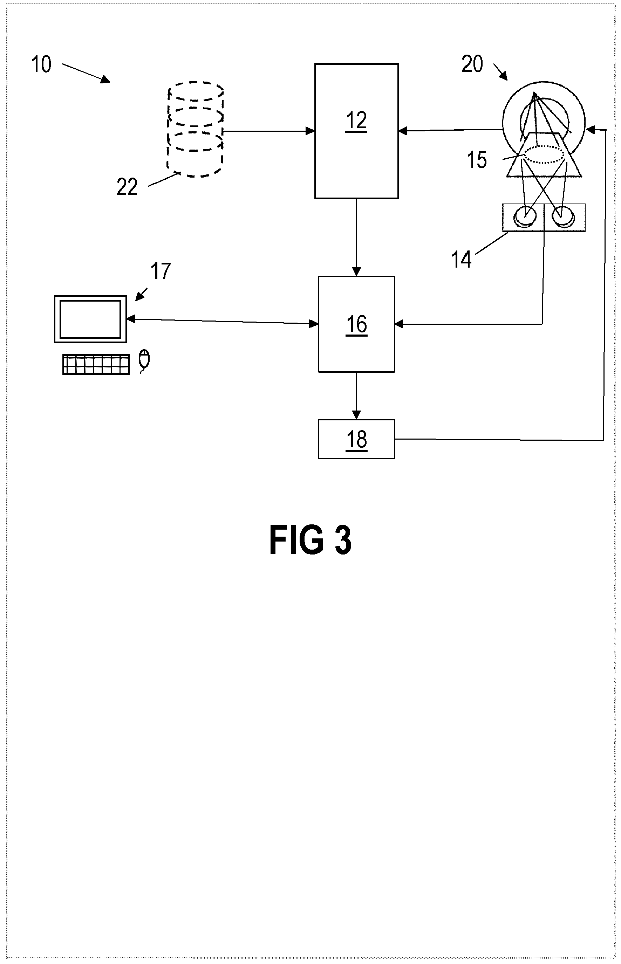

In a second aspect, the present invention relates to a system for planning an image acquisition of an object to be imaged by a tomographic imaging scanner. FIG 3 schematically shows an illustrative system in accordance with embodiments of the present invention.

Features, or details of the features, of a system in accordance with embodiments of the present invention shall be clear in view of the description provided hereinabove relating to a method in accordance with embodiments of the present invention.

The system 10 comprises an input 12 for receiving a pre-scan image of the object. Preferably, the pre-scan image may be a three-dimensional tomographic scan image, e.g. a 3D surview image, e.g. a low-dose 3D surview image.

The input may receive the pre-scan image from a storage device 22. The storage device may be comprised in the system, or may be external to the system.

The input may receive the pre-scan image from the (same) tomographic imaging scanner, which may be comprised in the system or may be external to the system.

The system 10 comprises a camera system 14 for capturing at least one image of the object. The camera system 14 may comprise an optical and/or infrared imaging system. The camera system 14 may comprise a range camera (such as a time-of-flight depth imaging system, a structured light imaging system, a lidar system, or the like). The camera system may comprise a plurality of cameras for imaging the object from a plurality of different viewpoints and a device to determine depth information from a plurality of images captured by the plurality of cameras. For example, the camera system may comprise a stereographic imaging system or a multi-camera depth imaging system. Thus, the at least one image may be an image comprising depth information, e.g. a three-dimensional image and/or a surface image with three-dimensional (non-planar) data describing points on the surface.

The system comprises a processor 16, e.g. a computer or other processing device suitable for performing the functions described further hereinbelow. The system may comprise executable instructions, e.g. stored in a memory, to perform these functions. The processor may be specifically designed in hardware, and/or programmed by software, to perform the functions described below.

The processor 16 is adapted to determine, e.g. configured for determining, corresponding image features in the at least one image captured by the camera system and in the pre-scan image. Determining corresponding image features may comprise detecting a plurality of surface features of the surface of the object in respectively the pre-scan image and the camera image, and determining corresponding pairs of the detected surface features between said images by comparing a local surface geometry of the body surface around each detected surface feature.

Furthermore, determining corresponding image features may comprise using a constellation model of the relative locations of the detected image features to identify and remove spurious detected features (or feature pairs).

Embodiments are not necessarily limited to a specific method of determining the corresponding image features, since many suitable approaches are known in the art, as the skilled person would appreciate. A more detailed description of suitable approaches is discussed hereinabove in relation to a method 100 in accordance with embodiments.

The processor 16 is adapted to determine an image transformation that relates the corresponding image features to each other, such that the object as represented by the pre-scan image can be transformed by the transformation (or equivalently by its inverse) to the orientation, position and/or deformation of the object represented in the camera image.

The processor 16 is adapted for automatically and/or interactively planning the tomographic image acquisition, in which the pre-scan image is used to determine parameters of the image acquisition that at least include a scan range in one dimension. For example the system may also comprise a user interface 17, e.g. comprising any one or more of a display monitor, a keyboard, a touch sensitive display, a digital pen and/or a pointer device (embodiments not limited to specific forms of human interface devices), to allow a user to interactively plan the image acquisition and/or to supervise and/or review an automatically generated plan.

This planning of the image acquisition comprises transforming the pre-scan image in accordance with the determined image transformation such that the position, orientation and/or deformation of the object in the pre-scan image corresponds to the position, orientation and/or deformation of the object as observed by the camera system. Additionally or alternatively, the planning of the image acquisition comprises transforming the determined parameters of the planned acquisition in accordance with the determined image transformation, such that the scan range as determined with respect to the pre-scan image is transformed to a same volume in the object as observed by the camera system.

The system comprises an output 18 to output the determined plan, e.g. in the form of instructions and/or signals that can be interpreted by the tomographic imaging scanner 20 to execute the planned imaging procedure. For example, the processor may be adapted to execute the planned image acquisition by controlling the tomographic imaging scanner.

The processor 16 may be adapted to repeatedly perform the steps of capturing the at least one image with the camera system, determining corresponding image features and determining the image transformation, in which the pre-scan image and/or the imaging plan are repeatedly updated by the most recently determined transformation (e.g. in a user interface presented to an operator of the system).

The processor 16 may be adapted for alerting an operator, e.g. using the user interface, when the determined transformation indicates a substantial movement of the object in the camera image (e.g. with respect to the pre-scan image).

The system may comprise a console system to operate the tomographic imaging scanner and/or a workstation to prepare an imaging plan for the tomographic imaging scanner.

The system may comprise the tomographic imaging scanner 20, in which the camera system may be configured in a predetermined imaging geometry with respect to the tomographic imaging scanner, e.g. such that spatial information in images captured by the camera system can be related to a coordinate system of the tomographic imaging scanner. The tomographic imaging scanner may comprise a computed tomography scanner or a magnetic resonance imaging scanner.

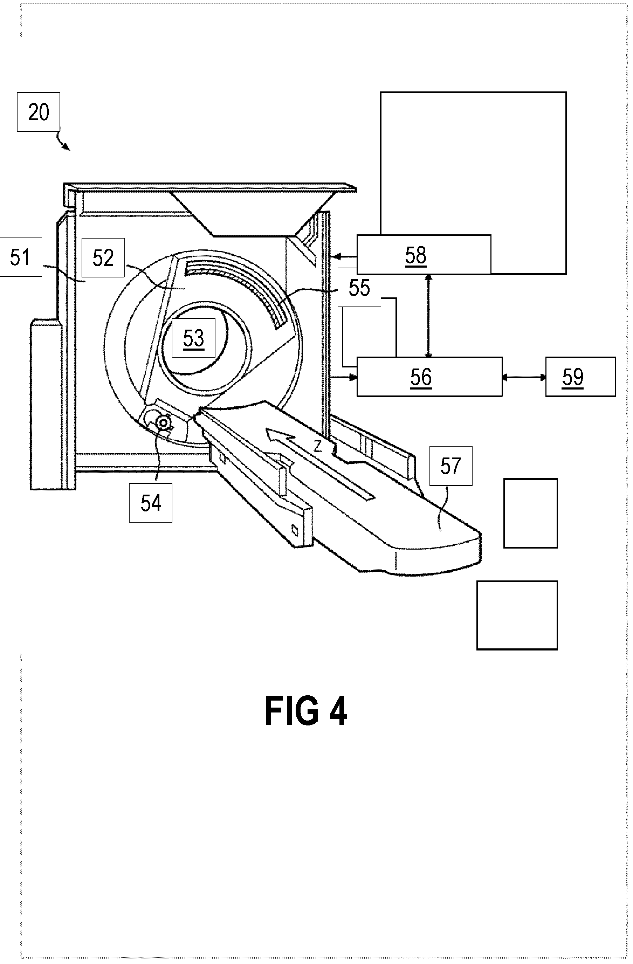

FIG 4 illustrates, schematically, an illustrative computed tomography scanner 20 as known in the art. The CT scanner may comprise a generally stationary gantry 51 and a rotating gantry 52. The rotating gantry 52 may be rotatably supported by the stationary gantry 51 and may rotate around an examination region 53 about a longitudinal axis Z.

A radiation source 54, such as an x-ray tube, may be rotatably supported by the rotating gantry 52, e.g. such as to rotate with this rotating gantry 52, and may be adapted for emitting poly-energetic radiation that traverses the examination region 53. The radiation source 54 may comprise, or consist of, a single broad spectrum x-ray tube. Alternatively, the radiation source may be adapted for controllably switching between at least two different photon emission spectra, e.g. switching between at least two different peak emission voltages, such as 80 kVp, 140 kVp, etc., during scanning. In another variation, the radiation source 54 may comprise two or more x-ray tubes configured to emit radiation with different mean spectrums.

A radiation sensitive detector array 55 may subtend an angular arc opposite the radiation source 54 across the examination region 53. The array 55 may include one or more rows of detectors arranged with respect to each other along the Z-axis direction. The array 55 may be adapted for detecting radiation traversing the examination region 53, and generating signals indicative thereof. The array 55 may comprise a detector with a plurality of pixelated radiation sensitive detector elements. Optionally, different detect elements may have different x-ray energy sensitivities, e.g. at least two scintillators and at least two corresponding photosensors having corresponding optical sensitivities and/or direct conversion detection elements (directly energy-resolving detector elements), e.g. such as allow a disambiguation of different (mean) photon energies to allow a spectral image detection. Additionally or alternatively, spectral information can be obtained by switching the radiation source 54 between different energy settings.

The system may comprise a reconstructor 56 for reconstructing the signals output by the detector array 55. The reconstructor 56 may be adapted for reconstructing tomographic, e.g. cross-sectional images (e.g. perpendicular to axis Z).

The system may comprise a subject support 57, such as a couch, for supporting an object or subject in the examination region. The system may also comprise an operator console 58, e.g. a general purpose computer programmed for controlling or monitoring the scanner system and/or for providing a user interface for an operator. The console may include a human readable output device such as a monitor or display and an input device such as a keyboard and mouse. Software resident on the console may allow the operator to interact with the scanner via a graphical user interface (GUI) or otherwise. This interaction may include selecting an imaging protocol, initiating scanning, etc.

The imaging system 59 may be operably connected to a workstation, e.g. computing system, such as a computer, that may comprise an input/output (I/O) interface for facilitating communication with the CT scanner. The scanner system may comprise the computing system as a system-level integrated component, or the imaging system may be adapted for communicating with a stand-alone computing system, e.g. to transmit image data to the computing system. The computing system may further comprise an output device. The output device or output devices may comprise, for example, a display monitor, a film printer, a paper printer and/or an audio output for audio feedback. The computing system may also comprise an input device or input devices, such as a mouse, a keyboard, a touch interface and/or a voice recognition interface. The computing system may also comprise at least one processor, such as a central processing unit (CPU), a microprocessor, a dedicated application-specific integrated circuit (ASIC) for processing and/or an appropriately configured programmable hardware processor such as a field-programmable gate array. The computing system may comprise a computer readable storage medium, e.g. a non-transitory memory such as a physical digital memory. The computer readable storage medium may store computer readable instructions and data. The at least one processor may be adapted for executing computer readable instructions. The at least one processor may also execute computer readable instructions carried by a signal, carrier wave or other transitory medium. Alternatively or additionally, the at least one processor may be physically configured to embody the instructions, e.g. entirely or in part, without necessarily requiring memory storage of these instructions, e.g. by configuration of a field-programmable gate array or an ASIC specifically designed to carry out at least a part of the instructions.

The computing system may be programmed, e.g. in accordance with the computer readable instructions comprising instructions to perform a method as disclosed hereinabove, to implement a system 10 in accordance with embodiments of the present invention.

In a third aspect, the present invention also relates to a computer program product, e.g. comprising the computer readable instructions referred to hereinabove, for performing a method in accordance with embodiments of the second aspect of the present invention, when executed on a computer. Embodiments of the present invention may also relate to data carrier or other non-transitory medium comprising said computer program product and/or to a transitory medium carrying the computer program product, e.g. a digital network transmission signal.

## Claims

1. A system (10) for planning a tomographic image acquisition of an object to be imaged by a tomographic imaging scanner (20), the system comprising:
an input (12) for receiving a pre-scan image of the object;
a camera system (14) for capturing at least one image of the object;
a processor (16), and
an output (18),
wherein said processor is adapted to:
determine corresponding image features in the at least one image captured by the camera system and the pre-scan image;
determine an image transformation that relates the corresponding image features to each other, such that the object as represented by the pre-scan image can be transformed by the transformation, or by its inverse, to the orientation, position and/or deformation of the object represented in the camera image;
automatically and/or interactively plan the tomographic image acquisition, in which the pre-scan image is used to determine parameters of the image acquisition that at least include a scan range in one dimension;
to output a signal, via said output, representative of the determined plan.

2. The system of claim 1, wherein said camera system (14) comprises a range camera and/or comprises a plurality of cameras for imaging the object from a plurality of different viewpoints and a device to determine depth information from a plurality of images captured by said plurality of cameras, such that the at least one image provided to the processor comprises three-dimensional information.

3. The system of any of the previous claims, comprising a user interface (17), operably connected to the processor (16), to enable a user, using said user interface, to interactively plan the image acquisition and/or to supervise and/or review an automatically generated plan.

4. The system of any of the previous claims, comprising a storage device (22), wherein said input (12) is adapted to retrieve the pre-scan image from the storage device.

5. The system of any of the previous claims, comprising the tomographic imaging scanner (20), wherein said camera system is configured in a predetermined imaging geometry with respect to the tomographic imaging scanner.

6. The system of claim 5, wherein said input (12) is operably connected to the tomographic imaging scanner to obtain the pre-scan image from the tomographic imaging scanner.

7. The system of claim 5 or claim 6, wherein said tomographic imaging scanner (20) comprises a computed tomography scanner or a magnetic resonance imaging scanner.

8. The system of any of the previous claims, comprising a console to operate the tomographic imaging scanner (20) and/or a workstation to prepare an imaging plan for the tomographic imaging scanner.

9. A method (100) for planning an image acquisition of an object to be imaged by a tomographic imaging scanner, the method comprising:
obtaining (110) a pre-scan image of the object;
capturing (120) at least one image of the object with a camera system that is configured in a predetermined imaging geometry with respect to the tomographic imaging scanner;
determining (130) corresponding image features in the at least one image captured by the camera system and pre-scan image;
determining (140) an image transformation that relates the corresponding image features to each other, such that the object as represented by the pre-scan image can be transformed by said transformation, or by its inverse, to the orientation, position and/or deformation of the object represented in the camera image, and
planning (150) the tomographic image acquisition, in which the pre-scan image is used to determine parameters of the image acquisition that at least include a scan range in one dimension,
wherein said planning of the image acquisition comprises:
transforming said pre-scan image in accordance with the determined image transformation such that the position, orientation and/or deformation of the object in the pre-scan image corresponds to the position, orientation and/or deformation of the object as observed by the camera system, and/or
transforming the determined parameters in accordance with the determined image transformation, such that said scan range as determined with respect to the pre-scan image is transformed to a same volume in the object as observed by the camera system.

10. The method of claim 9, wherein said capturing (120) of the at least one image comprises capturing an image comprising depth information, and wherein said obtaining (110) the pre-scan image comprises obtaining a three-dimensional tomographic surview scan image.

11. The method of claim 9 or 10, wherein said determining (130) corresponding image features comprises detecting a plurality of surface features of the surface of the object in respectively the pre-scan image and the camera image, and determining corresponding pairs of the detected surface features between said images by comparing a local surface geometry of the body surface around each detected surface feature.

12. The method of any of the claims 9 to 11, wherein said determining (130) corresponding image features comprises using a constellation model of the relative locations of the detected image features to identify and remove spurious detected features.

13. The method of any of the claims 9 to 12, comprising executing (160) the tomographic image acquisition using said tomographic imaging system in accordance with the determined parameters of said planning (150).

14. The method of any of the claims 9 to 13, wherein said steps of capturing (120) the at least one image with the camera system, determining (130) corresponding image features and determining (140) the image transformation are performed repeatedly, wherein the pre-scan image and/or the imaging plan are repeatedly updated by the most recently determined transformation.

15. A computer program product for performing a method in accordance with of any of the claims 9 to 14 when executed on a computer.
